(19) Europäisches Patentamt
European Patent Office
Office européen des brevets

(11) Numéro de publication : **0 451 004 A2**

(12) **DEMANDE DE BREVET EUROPEEN**

(21) Numéro de dépôt : **91400704.2**

(51) Int. Cl.$^5$ : **C07C 2/76**

(22) Date de dépôt : **15.03.91**

(30) Priorité : **27.03.90 FR 9004150**

(43) Date de publication de la demande : **09.10.91 Bulletin 91/41**

(84) Etats contractants désignés : **AT BE CH DE DK ES FR GB GR IT LI LU NL SE**

(71) Demandeur : **GAZ DE FRANCE**
**23, rue Philibert Delorme**
**F-75017 Paris (FR)**

(72) Inventeur : **Côme, Guy-Marie**
**9, rue W. Churchill**
**F-54000 Nancy (FR)**

(74) Mandataire : **Lerner, François et al**
**5, rue Jules Lefèbvre**
**F-75009 Paris (FR)**

(54) **Procédé et appareil de conversion d'hydrocarbures saturés.**

(57) L'invention concerne la conversion des hydrocarbures saturés.

Selon l'invention, on introduit dans une chambre (3) un premier gaz contenant du chlore et une deuxième gaz contenant de l'hydrogène, de manière à les mélanger. A la sortie (4) de cette chambre (3), on enflamme le mélange de chlore et d'hydrogène, et les produits issus de cette flamme sont alors mélangés dans une chambre (7) à un troisième gaz contenant les hydrocarbures à convertir, de teneur pondérale moyenne en élément hydrogène au moins égale à 18 %. Les effluents de la chambre (7) sont collectés, trempés et fractionnés. L'acide chlorhydrique est reformé en chlore qui est recyclé dans le premier gaz, l'hydrogène est recyclé dans le deuxième gaz, et les alcanes dans le troisième gaz. Les hydrocarbures insaturés sont récupérés.

L'invention s'applique en particulier à la conversion des gaz naturels en hydrocarbures insaturés, tel que l'éthylène.

EP 0 451 004 A2

FIG_1

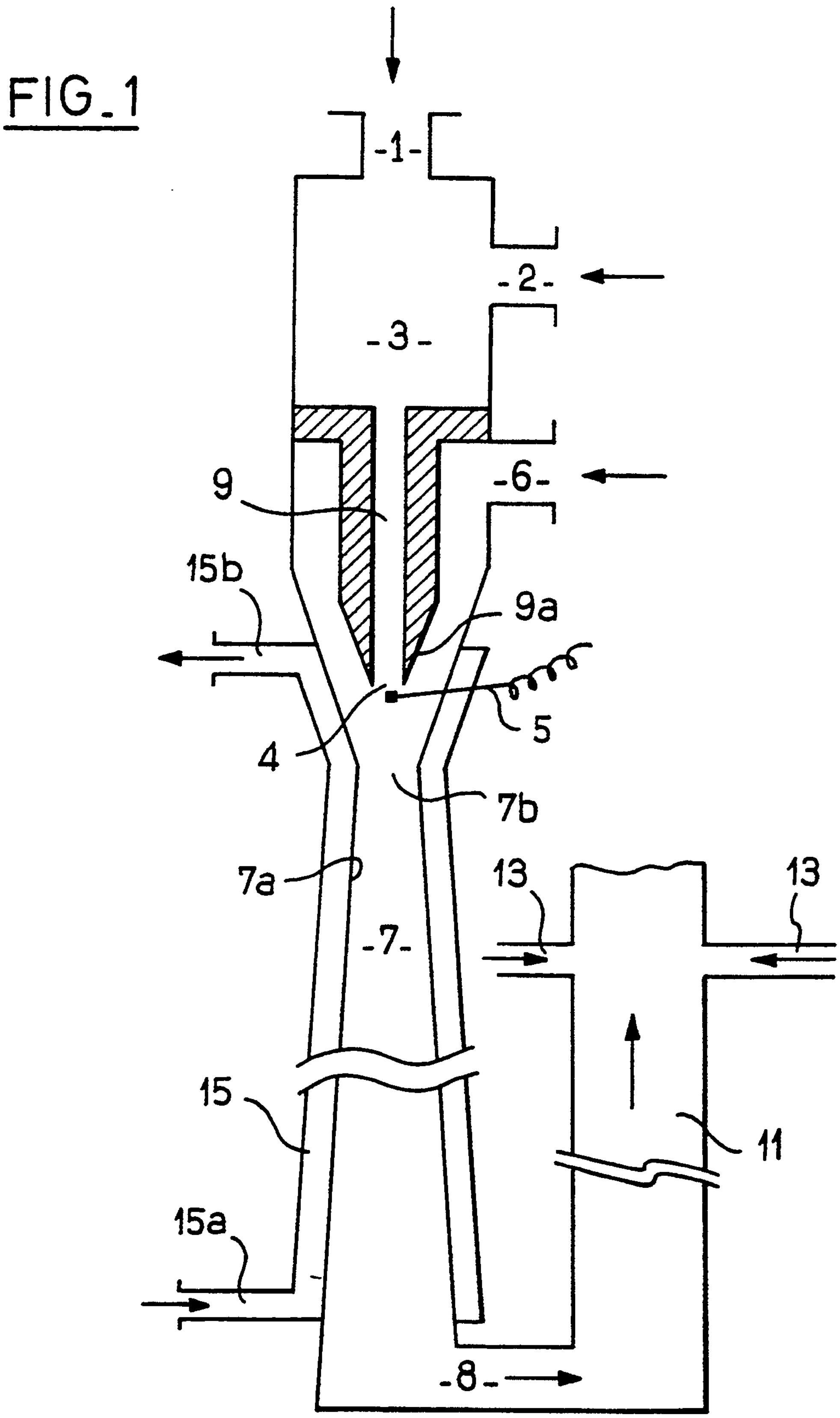

La présente invention se rapporte en particulier à un procédé de conversion d'hydrocarbures saturés, tels que le méthane, en hydrocarbures insaturés, tels que l'éthylène.

L'objectif visé est, de façon avantageuse, de convertir des matières premières hydrocarbonées en produits de base destinés en particulier aux industries chimiques.

Les charges hydrocarbonées pouvant être retenues dans le cadre de l'invention sont caractérisées par une teneur pondérale moyenne des hydrocarbures en élément hydrogène au moins égale à 18 % environ.

C'est le cas notamment de matières premières hydrocarbures gazeuses riches en méthane, telles que les gaz naturels et les gaz naturels synthétiques, certains gaz produits par gazéification de différents matériaux carbonés, comme le charbon, ou bien issus de la biomasse ou de différents déchets. C'est le cas aussi du méthane, de l'éthane, du propane et de leurs mélanges en toutes proportions.

Les butanes, les essences légères et naphtées, les fractions lourdes du pétrole ou issues de son raffinage et, d'une manière plus générale, les hydrocarbures gazeux ou liquides ayant une teneur moyenne pondérale en élément hydrogène inférieure à 18 % peuvent être utilisés dans le cadre de l'invention comme charges complémentaires, sous réserve de les mélanger à une autre charge d'hydrocarbures de teneur pondérale moyenne en élément hydrogène suffisamment supérieure à 18 % pour que la charge résultante ait une teneur pondérale moyenne en élément hydrogène supérieure à environ 18 %.

Les matières premières hydrocarbonées utilisées pour constituer les charges hydrocarbonées retenues dans le cadre de l'invention pourront contenir de petites quantités de gaz inorganiques et de divers composés soufrés ou oxygénés. L'élimination de ces corps des charges hydrocarbonées ne devrait pas être nécessaire dans le cadre de l'invention.

Quant aux produits de base qui sont obtenus par conversion de ces matières premières, et qui sont actuellement largement utilisés dans les industries chimiques, il s'agit d'hydrocarbures insaturés de faible poids moléculaire, tels que l'éthylène.

Il existe actuellement différents types de procédés de conversion des hydrocarbures.

En ce qui concerne les hydrocarbures ayant au moins deux atomes de carbone, c'est-à-dire à partir de l'éthane, le procédé le plus répandu est le vapocraquage. Ce procédé n'est pas applicable à des gaz riches en méthane.

En ce qui concerne ces gaz riches en méthane, ils sont surtout utilisés comme combustible, mais il existe également différents procédés pour convertir le méthane en produits chimiques de base, tels que le gaz de synthèse ou l'acétylène.

La conversion du méthane a été également obtenue par des procédés homogènes utilisant du chlore. Ces procédés peuvent être classés en deux grandes catégories.

Les procédés en deux stades consistent notamment à fabriquer d'abord du chlorure de méthyle par chloration du méthane à relativement basse température, puis à pyrolyser le chlorure de méthyle seul (brevet US-A-2 320 274), ou en présence d'oxygène (brevet US-A-4 714 796), en vue d'obtenir notamment des hydrocarbures insaturés. Ces procédés présentent l'inconvénient de conduire à la formation de goudrons et de suies, même si l'addition d'oxygène tend à réduire celle-ci.

Les procédés en un stade (brevet US-A-4 199 233 par exemple) consistent en particulier à faire réagir du méthane et du chlore dans une flamme à relativement haute température en vue de produire directement de l'éthylène et de l'éthane. Ces derniers procédés conduisent également à la formation de goudrons et suies.

L'invention a justement pour objet de résoudre les difficultés et inconvénients des différents procédés connus de conversion de gaz pouvant contenir du méthane, et surtout impliquant la mise en oeuvre de chlore.

Ce but est atteint dans l'invention en prévoyant :

- d'utiliser, en tant que substances de départ, un premier gaz contenant au moins de l'ordre de 30 % en volume de chlore, un deuxième gaz contenant au moins 30 % environ en volume d'hydrogène, et un troisième gaz contenant au moins 60 % environ en poids d'une charge d'un ou plusieurs hydrocarbures de teneur pondérale moyenne en élément hydrogène au moins égale à 18 % environ, et
- en une zone donnée de circulation des gaz, d'enflammer le chlore et l'hydrogène desdits premier et deuxième gaz alors mis en contact, en respectant une répartition spatiale telle que ces deux premiers gaz soient entourés par le troisième, et l'on fait se mélanger à ce troisième gaz les substances enflammées obtenues, de manière à induire les réactions de conversion des hydrocarbures.

En ce qui concerne la réaction exothermique entre le chlore et l'hydrogène, tant des flammes de prémélange que des flammes de diffusion peuvent être envisagées.

On notera par ailleurs, en ce qui concerne la charge d'hydrocarbures contenue dans le troisième gaz, qu'elle peut contenir des alcanes ou paraffines, incluant le méthane, des cyclanes, des cyclanoaromatiques, des aromatiques, voire des oléfines, à la seule condition que la teneur pondérale moyenne en élément hydrogène soit supérieure à, ou de l'ordre de, 18 %.

On notera également, en ce qui concerne les quantités volumiques de chlore, d'hydrogène et d'alcanes

(incluant le méthane) mises en jeu globalement dans les premier, second et troisième gaz, qu'un rapport chlore/alcanes inférieur ou égal à 1/1 et qu'un rapport hydrogène/chlore supérieur ou égal à 1/2, sont conseillés.

Les produits sortant du réacteur incluent des alcanes n'ayant pas réagi et/ou ceux formés lors de la réaction, des hydrocarbures insaturés, de l'hydrogène et de l'acide chlorhydrique.

Ces différents produits sont ensuite séparés les uns des autres par des procédés connus.

L'acide chlorhydrique est transformé en chlore par une réaction d'oxydation, selon l'équation :

$$2\ HCl + \frac{1}{2}\ O_2 \longrightarrow H_2O + Cl_2$$

Le chlore reformé peut être recyclé pour constituer le premier gaz de départ (en tout ou partie).

L'hydrogène collecté à la suite du fractionnement peut également être recyclé pour constituer au moins partiellement le deuxième gaz de départ.

Les paraffines collectées à la suite du fractionnement peuvent elles aussi être recyclées pour constituer au moins partiellement le troisième gaz de départ.

Les hydrocarbures insaturés issus de la section de fractionnement peuvent quant à eux être collectés et stockés pour une utilisation ultérieure.

Outre le procédé qui vient d'être présenté, l'invention a également pour objet un appareil ou réacteur de conversion permettant donc la conversion d'hydrocarbures saturés en hydrocarbures insaturés.

Selon une caractéristique importante de l'invention, cet appareil comprend :

- une chambre de réaction pour y mélanger et y faire réagir un premier gaz contenant du chlore, un second gaz contenant de l'hydrogène et un troisième gaz contenant une charge hydrocarbonée,
- des moyens d'allumage pour enflammer les premier et second gaz et induire la réaction de conversion en présence du troisième gaz,
- et des moyens de récupération communiquant avec la chambre de réaction pour collecter les effluents issus de cette chambre et contenant lesdits hydrocarbures insaturés.

Ainsi réalisé, cet appareil va permettre de mener à bien le procédé présenté ci-avant en obtenant les mêmes résultats avantageux en comparaison des appareils classiques utilisant notamment les procédés en un ou deux stade(s).

Selon une caractéristique complémentaire intéressante, cet appareil pourra présenter des chambres de préchauffage pour préchauffer au moins la charge hydrocarbonée à convertir, ces chambres s'étendant extérieurement au contact de la paroi extérieure de la chambre de réaction qui sera alors thermiquement conductrice, de manière à la fois à préchauffer cette charge hydrocarbonée avant de l'introduire dans la chambre de réaction et à créer dans cette dernière une zone de trempe interne par transfert de chaleur à travers ladite paroi conductrice de cette chambre.

De cette façon, la performance du réacteur et son coût de réalisation vont pouvoir être optimisés.

On notera que pour favoriser cette optimisation et pour des conditions pratiques de réalisation de l'appareil, il pourra être préférable de réaliser la chambre de réaction avec un rapport entre sa surface mouillée et son volume compris entre environ 40 et 250 $m^{-1}$, avantageusement avec une section rectangulaire.

D'autres objets, caractéristiques et avantages de l'invention pourront apparaître plus clairement dans la description qui va suivre faite en référence aux dessins d'accompagnement, dans lesquels :

- La figure 1 illustre schématiquement un premier mode de mise en oeuvre du procédé selon l'invention,
- La figure 2 illustre schématiquement un second mode de mise en oeuvre du procédé selon l'invention, et
- La figure 3 montre schématiquement une variante locale de réalisation de l'appareil présenté sur la figure 2.

Si l'on se reporte tout d'abord à la figure 1, on remarque que le premier gaz contenant du chlore est introduit par le conduit axial 1, le deuxième gaz contenant de l'hydrogène étant introduit par un conduit transversal 2. Ces deux gaz sont prémélangés dans une chambre 3 et enflammés à la sortie 4 de cette chambre par un dispositif d'allumage 5 connu en soi, (arc électrique, flamme auxiliaire,...), après être passés dans la tubulure en forme de col ou buse 9, de section sensiblement constante. Par le conduit 6 transversal le troisième gaz contenant la charge d'hydrocarbure(s) à convertir est introduit dans une chambre 7, et mélangé aux gaz de la flamme de prémélange issus de l'orifice 4, de manière à induire les réactions de conversion des hydrocarbures. Dans sa partie amont, la chambre 7 de mélange-réaction présente une partie convergente, un col, puis une longue partie aval divergente. Les effluents de la chambre 7 sont collectés par un conduit 8 pour être trempés et fractionnés par des procédés connus en soi.

Après fractionnement,

– l'acide chlorhydrique est converti en chlore dans une installation auxiliaire par un procédé connu en soi, et le chlore est recyclé vers le conduit 1,
– l'hydrogène est recyclé vers le conduit 2,
– les alcanes sont recyclés vers le conduit 6,
– les hydrocarbures insaturés sont collectés et stockés pour une utilisation ultérieure.

Pour éviter de surcharger la figure 1, on n'a pas représenté les conduits de recyclage.

On a observé que le rendement du procédé en produits de transformation valorisables est amélioré par un préchauffage notamment du troisième gaz contenant la charge d'hydrocarbures. Ce préchauffage peut être réalisé par différents procédés , par exemple par échange de chaleur à travers les parois de la chambre 7, ou par récupération de la chaleur latente des gaz issus du conduit 8 par l'intermédiaire d'un fluide de trempe ou par récupération de la chaleur dégagée par l'oxydation de l'acide chlorhydrique, ou encore par chauffage de la charge d'hydrocarbures en utilisant la chaleur dégagée par la combustion soit d'une fraction de la charge elle-même soit d'un combustible auxiliaire, voire par chauffage par de l'énergie électrique dégradée, étant entendu que ces moyens peuvent être mis en oeuvre séparément ou concomitamment, en tout ou en partie. En 11, à la sortie du conduit 8, on a d'ailleurs représenté une colonne classique de trempe "directe", c'est-à-dire de trempe par injection ou introduction, via les conduits 13, d'un fluide de trempe. En tant que tel fluide de trempe, on peut envisager d'utiliser de l'hydrogène ou un hydrocarbure et en particulier un hydrocarbure "léger" (contenant moins de cinq atomes de carbone), de manière à induire une trempe dite réactive.

Bien entendu d'autres types de trempe pourraient être utilisés tels qu'une trempe par un liquide, par exemple injecté en partie basse de la chambre 7.

Selon une caractéristique importante de l'invention, on pourrait également prévoir une trempe "indirecte" des produits circulant dans la chambre de conversion 7.

Les parois 7a qui limitent extérieurement cette chambre seront alors thermiquement conductrices. Leur réalisation métallique (notamment du Nickel , métal résistant au chlore) ou en céramiques est tout à fait envisageable et procure même des avantages appréciables.

On peut en particulier alors conjuguer la trempe des produits de conversion circulant dans la chambre 7 avec le préchauffage de tout ou partie des réactifs, et notamment de la charge hydrocarbonée circulant alors (de préférence à contre-courant) dans une ou plusieurs chambre(s) de préchauffage telle que 15 s'étendant autour de la chambre 7, le long de celle-ci, au contact de ses parois extérieures 7a pour autoriser le transfert de chaleur.

Pour obtenir des résultats satisfaisants pour cette action combinée et concomitante trempe indirecte/préchauffage, il est toutefois conseillé de prévoir un rapport entre la surface mouillée des parois de la chambre 7 et le volume de cette même chambre compris entre 40 et 250 $m^{-1}$. Ces limites s'expliquent d'un côté par des problèmes d'efficacité des échanges thermiques (qui ne sont pas suffisants en-dessous de $40 m^{-1}$) et de l'autre par des difficultés de réalisation industrielle liées en particulier à la constitution matérielle des parois de la chambre, à l'écartement minimum entre ces parois autorisant une circulation des réactifs, à l'épaisseur des lèvres 9a du brûleur....

Compte tenu de ces exigences, une réalisation de cette chambre 7 avec une section rectangulaire devrait être préférée (dans ce cas au détriment d'une section habituellement circulaire).

Pour une efficacité optimale, l'entrée 15a de la (des) chambre(s) de préchauffage 15 pourra être située vers la base de la chambre de conversion 7, le (s) réactif(s) à préchauffer circulant ensuite à contre-courant des produits contenus dans le réacteur, pour ressortir en 15b au niveau de la partie supérieure de cette chambre 7, à proximité du col 7b de celle-ci.

La température des gaz à l'intérieur de la chambre 7 est difficile à préciser et dépend des conditions opératoires, en particulier de la température de préchauffage éventuelle de la charge d'hydrocarbures, de la nature de cette charge, des taux de chlore et d'hydrogène mis en jeu. Les températures de conversion des hydrocarbures devraient être en partie amont de la chambre 7 comprises entre 700 et 1600°C. Grâce à la répartition spatiale particulière des différents gaz à leur arrivée dans la chambre 7, le troisième gaz contenant les hydrocarbures à pyrolyser sert d'isolant thermique entre la flamme issue de l'orifice 4 et les parois de la partie amont de la chambre 7, ce qui assure une diminution des contraintes thermiques dans le réacteur.

La réaction peut être conduite à une pression proche de la pression atmosphérique, mais elle peut être également conduite à des pressions plus basses, jusqu'à environ $0,5 \times 10^5$ Pa ou plus élevées, jusqu'à environ $50 \times 10^5$ Pa.

Les temps de passage des corps dans la chambre de réaction 7 seront en général inférieurs à 1 s.

Intéressons-nous maintenant à la figure 2 pour voir illustrée une variante de réalisation de l'invention.

Dans cette variante, la chambre de réaction 7 qui présente une section rectangulaire sensiblement constante est raccordée en partie supérieure à une série de veines ou conduits 19, 21, 23 d'alimentation en réactifs. Ces différentes veines s'étendent, à proximité de la chambre, suivant des axes sensiblement parallèles à la

direction longitudinale 17 de cette chambre.

En l'espèce le conduit central 19 est prévu pour alimenter la chambre 7 en chlore ou en un mélange de chlore et d'hydrogène. Le second conduit 21 pourrait à la limite être omis. Lorsqu'il est prévu ce conduit est utilisé pour alimenter la chambre en hydrogène, ceci donc essentiellement dans le cas où cet hydrogène n'est apporté ni par le premier (19) ni par le troisième conduit 23, ce dernier servant donc à alimenter la chambre 7 soit en un mélange d'hydrocarbures saturés à convertir et d'hydrogène, soit en hydrocarbures saturés seuls. En ce qui concerne la nature des constituants, celle-ci est toujours la même, la chambre devant recevoir, du centre vers l'extérieur du réacteur, essentiellement du chlore, de l'hydrogène et la charge hydrocarbonée.

Si trois telles veines sont prévues, un premier gaz contenant au moins 30 % en volume de chlore circulera donc dans la première veine centrale 19, un second gaz contenant au moins 30 % environ en volume d'hydrogène circulera alors dans la seconde veine 21 et un troisième gaz contenant au moins 60 % environ en poids d'une charge hydrocarbonée de teneur pondérale moyenne en élément hydrogène au moins égale à environ 18 % circulera dans la troisième veine 23 extérieure .

Si toutefois dans cette troisième veine circule un mélange d'hydrogène et de charge hydrocarbonée, ce composé "prémélangé" présentera de préférence une quantité en volume d'hydrogène comprise entre 1 et 3 fois la quantité en volume de charge hydrocarbonée, ce rapport étant avantageusement de l'ordre de 2.

Sur la figure 2, le dispositif d'allumage 5 déjà présenté permet d'allumer une flamme de diffusion entre le chlore et l'hydrogène contenus dans les premier et second gaz.

Les produits de cette flamme de diffusion se mélangent alors au troisième gaz riche en hydrocarbures qui entourent ces produits,déclenchant ainsi la réaction de conversion de la charge hydrocarbonée.

A l'issue, les produits de conversion et plus généralement les effluents sont récupérés dans les conduits de récupération 25 situés en l'espèce à la base de la chambre 7, éventuellement après trempe.

Comme dans la version de la figure 1, il pourra s'avérer avantageux de réaliser les parois extérieures 7a de cette chambre 7 en matériau thermiquement conducteur et de prévoir pour cette même chambre un rapport surface mouillée/volume compris entre environ 40 et 250 $m^{-1}$ pour pouvoir réaliser simultanément une trempe indirecte des effluents et un préchauffage de tout ou partie des réactifs dans les chambres de préchauffage 15 qui s'étendent ici au contact et le long desdites parois 7a, entre, en partie supérieure, le niveau où les gaz sont enflammés et où la réaction de conversion est initiée et, en partie inférieure, l'endroit où la chambre 7 s'élargit, via le divergent 27.

De façon complémentaire, cette première trempe "indirecte" pourrait être suivie d'une seconde trempe directe menée par exemple à la sortie des conduits 25 dans une colonne de trempe telle que celle repérée 11 sur la figure 1 et présentée ci-avant.

Dans le cas de la figure 2, on aura remarqué que l'on se trouve dans le cas typique d'un réacteur de conversion à flamme de diffusion chlore/hydrogène, les veines d'alimentation s'interrompant ici à l'entrée de la chambre 7 sensiblement à un même niveau.

On pourrait toutefois préférer, comme illustré sur la figure 3, étager l'admission des réactifs.

Sur cette figure 3, trois veines sensiblement "concentriques" et parallèles 31, 33, 35 ont été prévues et sont alimentées respectivement, de l'intérieur vers l'extérieur du réacteur, essentiellement en chlore, hydrogène et charge hydrocarbonée.

Le premier conduit central 31 d'alimentation en chlore s'interrompt le premier, définissant ainsi une première chambre de réaction 37 limitée approximativement en aval par l'interruption du second conduit 33 d'alimentation en hydrogène et qui entoure le premier. Le dispositif d'allumage 39 situé dans la chambre 37 permet d'allumer une flamme de diffusion entre le premier gaz riche en chlore et le second gaz riche en hydrogène.

A la sortie de la chambre 37, les produits de la flamme de diffusion reçoivent autour d'eux le troisième gaz contenant la charge hydrocarbonée à convertir qui débouche du conduit le plus extérieur 35, en l'espèce juste en amont de l'entrée de la chambre 7 définie ici par son col d'admission 7b.

On va maintenant présenter deux exemples non limitatifs à titre d'illustration de l'invention.

<u>Exemple 1</u>

. Le réacteur utilisé est du type illustré sur la figure 1.

. On alimente le conduit 1 par du chlore, le conduit 2 par de l'hydrogène, et le conduit 6 par du méthane préchauffé à 500°C environ après passage dans les chambres de préchauffage 15. Les trois gaz sont dans les proportions volumiques respectives 1/0,8/1. A la sortie du conduit 4, l'inflammation est amorcée par le dispositif 5, et la réaction de conversion a lieu dans la chambre de réaction 7. La pression dans le réacteur est un peu supérieure à la pression atmosphérique. Le mélange de gaz collecté par le conduit 8 contient essentiellement du mélange non réagi, de l'éthane, de l'éthylène, de l'acétylène, de l'hydrogène et de l'acide chlorhydrique, voire peut être du benzène.

Exemple 2

. Le réacteur utilisé est du type schématisé sur la figure 2.

. On alimente le conduit 19 par du chlore, le conduit 21 par de l'hydrogène, et le conduit 23 par un gaz naturel contenant environ 76 % de méthane en volume, 12 % d'éthane, 8 % de propane, 2 % de butanes et de petites quantités de divers gaz organiques et inorganiques. Le chlore, l'hydrogène et le gaz naturel sont dans des proportions volumiques respectives de 0,9/0,8/1. Le gaz naturel a été préchauffé à 500 °C environ. Après mise en oeuvre de la réaction à une pression proche de l'atmosphère, on recueille les gaz issus du conduit 25. Le mélange collecté contient essentiellement du méthane, de l'éthane, de l'éthylène, du propène, de l'hydrogène et de l'acide chlorhydrique, ainsi que de l'acétylène, du propane, des butanes, des butènes et du butadiène.

## Revendications

**1.** - Procédé de conversion d'hydrocarbures saturés, tels que du méthane, en hydrocarbures insaturés, tels que l'éthylène, caractérisé en ce que :

a) on utilise, en tant que substances de départ, un premier gaz contenant au moins 30 % environ en volume de chlore, un deuxième gaz contenant au moins 30 % environ en volume d'hydrogène, et un troisième gaz contenant au moins 60 % environ en poids d'une charge d'un ou plusieurs hydrocarbures de teneur pondérale moyenne en élément hydrogène au moins égale à environ 18%, et

b) en une zone donnée de circulation des gaz, on enflamme le chlore et l'hydrogène desdits premier et deuxième gaz alors mis en contact, en respectant une répartition spatiale telle que ces deux premiers gaz soient entourés par le troisième, et l'on fait se mélanger à ce troisième gaz les substances enflammées obtenues, de manière à induire les réactions de conversion des hydrocarbures.

**2.** - Procédé selon la revendication 1 caractérisé en ce que la répartition spatiale des gaz est telle qu'on fait circuler au centre le premier gaz, entouré par le second, lui-même entouré par le troisième.

**3.** - Procédé selon la revendication 1 ou la revendication 2 caractérisé en ce que avant d'enflammer le chlore et l'hydrogène des premier et deuxième gaz on mélange entre eux ces gaz, sans qu'il y ait mélange avec le troisième gaz.

**4.** - Procédé selon la revendication 1 ou la revendication 2 caractérisé en ce qu'avant d'enflammer le chlore et l'hydrogène des premier et second gaz, on fait circuler des gaz non mélangés pour obtenir lors de l'étape b) de la revendication 1 sensiblement une flamme de diffusion entre le chlore et l'hydrogène.

**5.** - Procédé selon l'une des revendications 1 à 4, caractérisé en ce que la quantité volumique de chlore contenue dans le premier gaz est inférieure ou égale à la quantité volumique d'alcanes contenue dans le troisième gaz.

**6.** - Procédé selon l'une quelconque des revendications précédentes, caractérisé en ce que la quantité volumique d'hydrogène contenue dans le deuxième gaz est au moins égale à la moitié de la quantité volumique de chlore contenue dans le premier gaz.

**7.** - Procédé selon l'une quelconque des revendications précédentes, caractérisé en ce qu'après l'étape b) de la revendication 1 et après trempe des effluents issus des réactions de conversion, on sépare l'acide chlorhydrique des produits de conversion, on reforme du chlore à partir de l'acide chlorhydrique obtenu, et on recycle le chlore reformé pour constituer au moins partiellement le premier gaz de départ.

**8.** - Procédé selon l'une quelconque des revendications précédentes, caractérisé en ce qu'après l'étape b) de la revendication 1 et après trempe des effluents issus des réactions de conversion, on sépare l'hydrogène des produits de conversion et on recycle cet hydrogène pour constituer au moins partiellement le deuxième gaz de départ.

**9.** - Procédé selon l'une quelconque des revendications précédentes, caractérisé en ce qu'après l'étape b) de la revendication 1 et après trempe des effluents issus des réactions de conversion, on sépare les alcanes des autres produits de conversion et on les recycle pour constituer au moins partiellement le troisième gaz de départ.

**10.** - Procédé selon l'une quelconque des revendications précédentes, caractérisé en ce qu'on effectue un préchauffage au moins du troisième gaz contenant la charge d'hydrocarbure(s) à convertir.

**11.** - Procédé selon la revendication 10, caractérisé en ce qu'à l'issue de l'étape b) de la revendication 1, pour tremper les effluents issus des réactions de conversion, on conduit un échange thermique indirect, sans qu'il y ait mélange, entre lesdits produits circulant dans la chambre de conversion et une partie au moins desdites substances de départ que l'on fait circuler autour des produits de conversion, de manière à assurer concomitamment ladite trempe et le préchauffage de ces substances de départ en circulation.

**12.** - Procédé selon l'une quelconque des revendications précédentes, caractérisé en ce que la réaction de conversion est conduite à une pression comprise entre 0,5 x $10^5$ Pa et 50 x $10^5$ Pa.

**13.** - Procédé selon l'une quelconque des revendications précédentes, caractérisé en ce que la réaction de conversion se déroulant dans une chambre de conversion, le temps de passage des substances dans cette chambre est inférieur à 1 s.

**14.** - Appareil pour la conversion d'hydrocarbures saturés en hydrocarbures insaturés comprenant :

– une chambre de réaction pour y mélanger et y faire réagir un premier gaz contenant du chlore, un second gaz contenant de l'hydrogène et un troisième gaz contenant une charge hydrocarbonée à convertir,

– des moyens d'allumage pour enflammer les premier et second gaz et induire les réactions de conversion en présence du troisième gaz,

– et des moyens de récupération communiquant avec la chambre de réaction pour collecter les effluents issus de cette réaction et contenant lesdits hydrocarbures insaturés.

**15.** - Appareil selon la revendication 14 caractérisé en ce qu'il comprend en outre une chambre de prémélange où lesdits premier et second gaz sont mélangés avant d'alimenter la chambre de réaction, de manière à obtenir dans cette dernière une flamme de prémélange, en présence de la charge hydrocarbonée.

**16.** - Appareil selon la revendication 15 caractérisé en ce qu'il comprend un conduit pour alimenter la chambre de réaction avec ledit troisième gaz, ce conduit amenant ce troisième gaz autour d'une buse à travers laquelle le mélange des premier et second gaz est amené dans ladite chambre de réaction.

**17.** - Appareil selon la revendication 14 caractérisé en ce qu'il comprend deux conduits pour alimenter en gaz la chambre de réaction, ces conduits comprenant :

– un premier conduit où circule le chlore ou un mélange contenant du chlore et de l'hydrogène destiné à alimenter la chambre de réaction,

– et un second conduit s'étendant extérieurement, autour du premier, et sensiblement parallèlement à ce dernier, dans ce second conduit circulant la charge hydrocarbonée ou un mélange contenant une telle charge et de l'hydrogène destiné(e) à alimenter ladite chambre de réaction,

– ces deux conduits s'étendant sensiblement parallèlement à une direction longitudinale de la chambre de réaction, au moins à proximité de cette dernière avec laquelle ils communiquent.

**18.** - Appareil selon la revendication 17 caractérisé en ce qu'il comprend en outre un troisième conduit s'étendant entre les deux premiers conduits, sensiblement parallèlement à eux, dans ce troisième conduit circulant de l'hydrogène destiné à alimenter ladite chambre de réaction.

**19.** - Appareil selon l'une quelconque des revendications 13 à 18 caractérisé en ce qu'il comprend en outre des chambres de préchauffage pour préchauffer au moins ladite charge hydrocarbonée, ces chambres s'étendant extérieurement au contact de la paroi extérieure de la chambre de réaction qui est thermiquement conductrice, de manière à la fois à préchauffer cette charge hydrocarbonée avant de l'introduire dans ladite chambre de réaction et à créer dans cette dernière une zone de trempe interne par transfert de chaleur de la réaction entre le chlore et l'hydrogène vers lesdites chambres de préchauffage, à travers ladite paroi conductrice de ladite chambre de réaction.

**20.** - Appareil selon l'une quelconque des revendications 14 à 19 caractérisé en ce que la chambre de réaction présente une section rectangulaire.

**21.** - Appareil selon l'une quelconque des revendications 14 à 20 caractérisé en ce que la chambre de réaction présente un rapport entre la surface mouillée de ses parois et son volume compris entre environ 40 et 250$m^{-1}$

**22** - Appareil selon l'une quelconque des revendications 18 à 21 caractérisé en ce que les trois conduits d'alimentation en gaz de la chambre de réaction se terminent à des niveaux différents, de manière à enflammer les premier et deuxième gaz avant de mettre ces gaz enflammés au contact du troisème gaz.

FIG_1

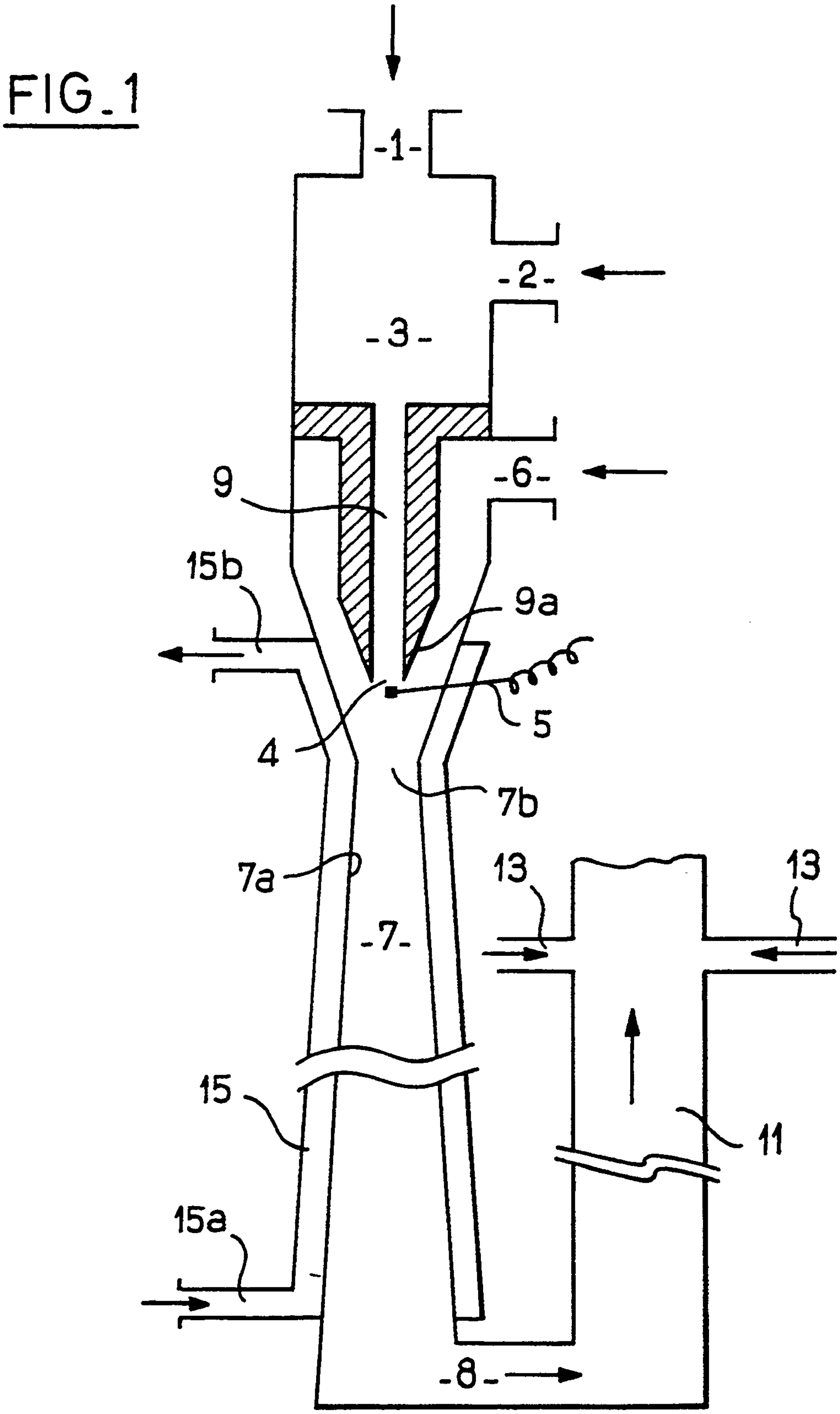

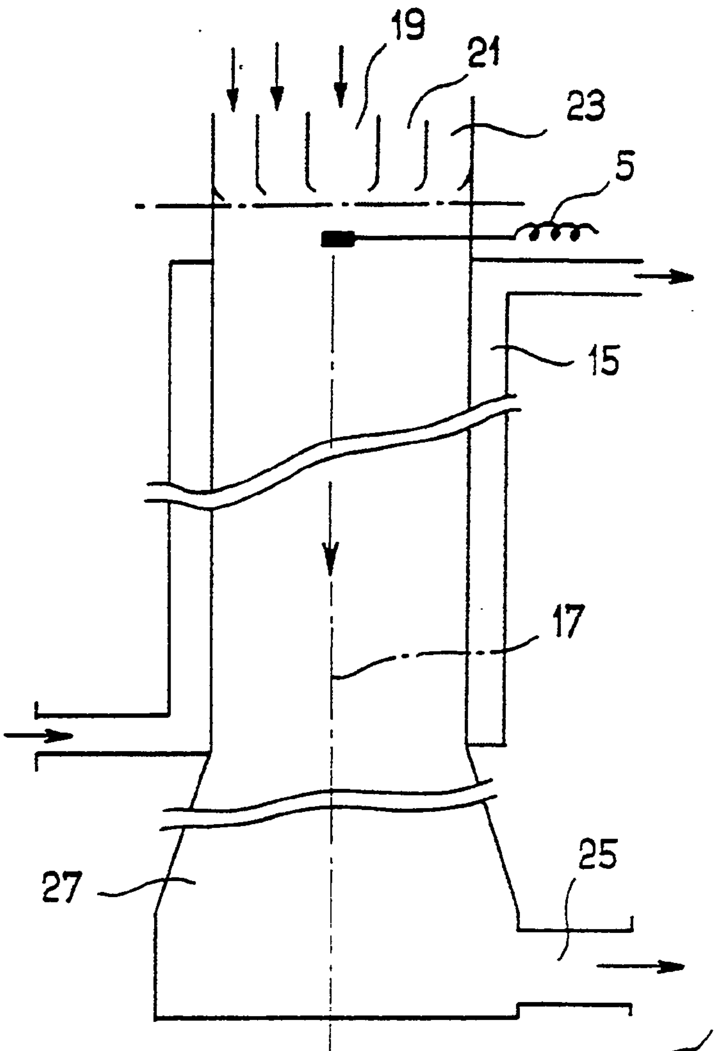


FIG_2

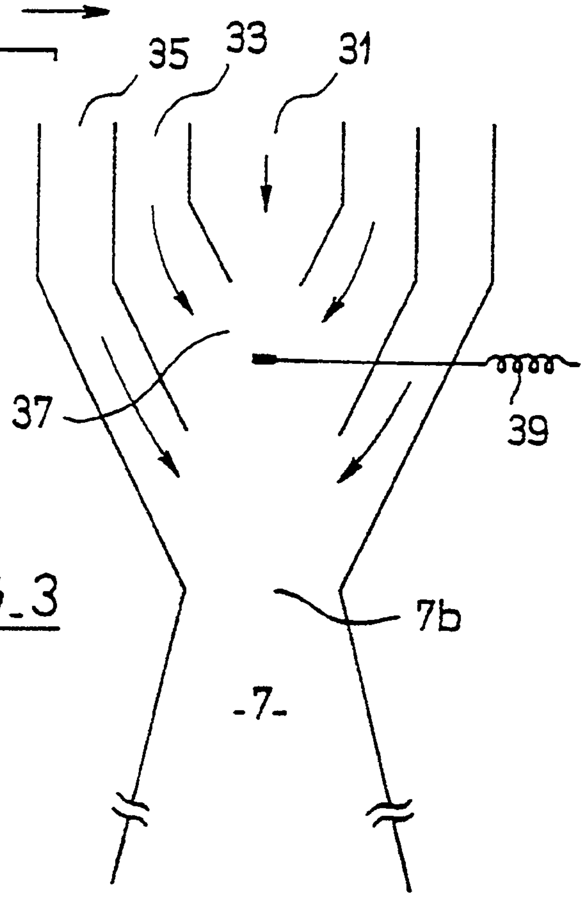


FIG_3